# EUROPÄISCHE PATENTSCHRIFT

(11) **EP 0 601 413 B1**
(45) Veröffentlichungstag und Bekanntmachung des Hinweises auf die Patenterteilung: **26.01.2000**
(21) Anmeldenummer: 93119087.0
(22) Anmeldetag: 26.11.1993
(51) Int. Cl.: C07F 9/53, C08F 2/50, G03F 7/029, A61K 6/083, C07F 9/6571, C07F 9/40, C07F 9/32

(54) **Verfahren zur Herstellung von Arenbisphosphinoxide**
Method for the productin of Arene bisphosphinoxides
Procedure pour la preparation des Bisphosphineoxydes arèniques

(30) Priorität: 05.12.1992 DE 4240964
(43) Veröffentlichungstag der Anmeldung: 15.06.1994
(73) Patentinhaber: BASF Aktiengesellschaft, 67063 Ludwigshafen (DE)
(72) Erfinder: Schroeder, Jochen, Dr., D-6703 Limburgerhof (DE); Siegel, Wolfgang, Dr., D-6800 Mannheim 1 (DE); Lokai, Matthias, D-6753 Enkenbach-Alsenborn (DE)

(56) Entgegenhaltungen:
- EP-A- 0 007 508
- EP-A- 0 073 413
- DE-A- 2 245 817
- DE-B- 1 010 965
- K. Sasse in Houben-Weyl, Band 12/1, S. 208-209, G. Thieme-Verlag, Stuttgart
- Weygand-Hilgetag, Organisch-Chemische Experimentierkunst, 4. Auflage, S. 246-256, J. A. Barth-Verlag, Leipzig 1970

## Beschreibung

Die Erfindung betrifft ein Verfahren zur Herstellung von Arenbisphosphinoxiden der allgemeinen Formel 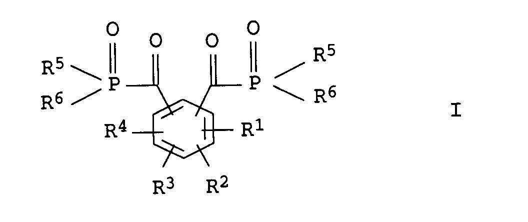 worin die Reste R¹ bis R⁴ unabhängig voneinander für ein Wasserstoffatom oder eine C₁-C₆-Alkyl-, -Alkoxygruppe oder eine C₅-C₁₂-Aryl-, eine C₅-C₁₂-Aryloxy- oder eine C₇-C₁₄-Arylalkoxygruppe stehen, R⁵ und R⁶ unabhängig voneinander für eine C₁-C₆-Alkylgruppe, eine C₅-C₁₀-Cycloalkylgruppe oder eine C₅-C₁₂-Arylgruppe, welche auch ein oder zwei Stickstoff- oder Schwefelatome im Ringsystem enthalten oder ein oder zwei Halogenatome, C₁-C₆-Alkyl- oder -Alkoxygruppen als Substituenten am Ringsystem tragen können, oder für eine C₁-C₆-Alkoxy-, eine C₃-C₈-Alkyloxyalkoxy-, eine C₅-C₁₂-Aryloxy- oder C₇-C₁₄-Arylalkoxygruppe stehen können oder R⁵ und R⁶ gemeinsam eine Brücke aus 2 bis 10 Kohlenstoffatomen bilden, wobei auch ein Teil der Kohlenstoffatome Bestandteil eines aromatischen Rings sein kann.

Weiterhin betrifft die Erfindung photopolymerisierbare Massen, welche die Arenbisphosphinoxide enthalten und die Verwendung dieser Massen als Beschichtungsmassen, z.B. als Lack oder Druckfarbe, als Füll- oder Spachtelmasse, als Dentalmasse, als Klebstoff oder zur Herstellung von Formkörpern, z.B. Druckplatten oder Reliefformen.

Bei bisher bekannten Photoinitiatoren für die Polymerisation ethylenisch ungesättigter Verbindungen handelt es sich z.B. um aromatische Ketone wie Acetophenon- und Benzophenon-Derivate, Thioxanthone, Benzoinether sowie Benzilketale. Mit derartigen Initiatoren ausgehärtete Massen zeigen jedoch eine unerwünschte Vergilbung, die eine Verwendung unter Zusatz von weißen Pigmenten nicht zuläßt. Ein weiterer Nachteil ist die geringe Dicke der ausgehärteten Schicht.

Eine Verbesserung konnte mit Acylphosphinoxiden, wie sie z.B. in der EP-B-57 474 oder EP-A-73 413 beschrieben sind, erreicht werden.

Die aus der EP-A-184 095 bekannten Bisacylphosphinoxide sind in photopolymerisierbaren Massen nur unzureichend löslich und daher nur schwer verarbeitbar.

Aus EP-A-7508 sind Acylphosphinoxide und Arenbisphosphinoxide sowie ihre Verwendung als Photoinitiatoren bekannt.

Generell besteht ein Bedarf an möglichst reaktiven Photoinitiatoren.

Mit reaktiveren Photoinitiatoren kann die für eine Härtung ausreichende Zusatzmenge zu photopolymerisierbaren Massen verringert werden. Insbesondere erhöht sich mit zunehmender Reaktivität auch die Aushärtungsgeschwindigkeit. Dieser Vorteil ist von besonderer Bedeutung bei der heute üblichen Produktionsweise, bei der die photopolymerisierbaren Massen auf einem Transportband liegend unter einer Strahlenquelle durchgeführt werden. Bei kürzeren Härtungszeiten kann die Geschwindigkeit des Transportbandes erhöht und so der Durchsatz gesteigert werden.

Aufgabe der vorliegenden Erfindung war daher ein Verfahren zur Herstellung von Photoinitiatoren, welche eine möglichst hohe Reaktivität besitzen und nicht zu einer Vergilbung der ausgehärteten Massen führen.

Demgemäß wurde ein Verfahren zur Herstellung von Arenbisphosphinoxiden der allgemeinen Formel I, 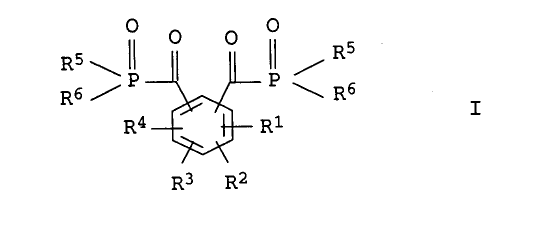 worin die Reste R¹ bis R⁴ unabhängig voneinander für ein Wasserstoffatom oder eine C₁-C₆-Alkyl-, -Alkoxygruppe oder eine C₅-C₁₂-Aryl-, eine C₅-C₁₂-Aryloxy- oder eine C₇-C₁₄-Arylalkoxygruppe stehen, R⁵ und R⁶ unabhängig voneinander für eine C₁-C₆-Alkylgruppe, eine C₅-C₁₀-Cycloalkylgruppe oder eine C₅-C₁₂-Arylgruppe, welche auch ein oder zwei Stickstoff- oder Schwefelatome im Ringsystem enthalten oder ein oder zwei Halogenatome, C₁-C₆-Alkyl- oder -Alkoxygruppen als Substituenten am Ringsystem tragen können, oder für eine C₁-C₆-Alkoxy-, C₃-C₈-Alkyloxyalkoxy-, eine C₅-C₁₂-Aryloxy- oder C₇-C₁₄-Arylalkoxygruppe stehen können oder R⁵ und R⁶ gemeinsam eine Brücke aus 2 bis 10 Kohlenstoffatomen bilden, wobei auch ein Teil der Kohlenstoffatome Bestandteil eines aromatischen Rings sein kann, gekennzeichnet durch folgende Verfahrensschritte gefunden:
a) Zweifache Halogenacetylierung einer Verbindung der Formel 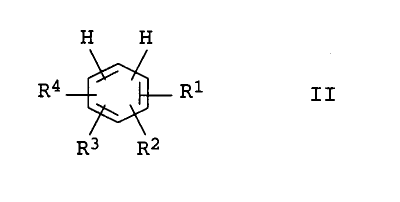 mit α-Halogenacetylhalogeniden in Gegenwart von Eisen-III-oxid.
b) Überführung des in a) erhaltenen Bishalogenacetophenon der Formel 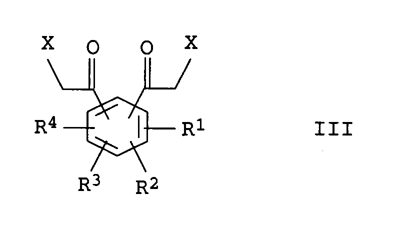 durch Umsetzung mit einer Alkalihypohalogenidlösung in Gegenwart eines Phasentransferkatalysators in die Dicarbonsäure der Formel 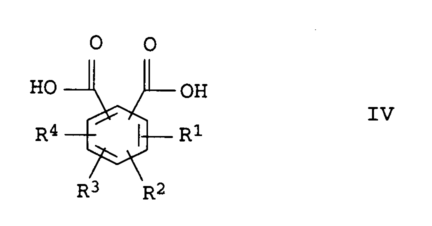
c) Überführung der Dicarbonsäure in das entsprechende Bissäurechlorid der Formel 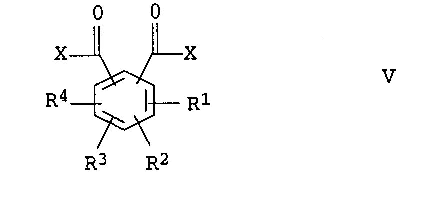 und
d) Umsetzung des Bissäurechlorids mit Phosphinen der Formel 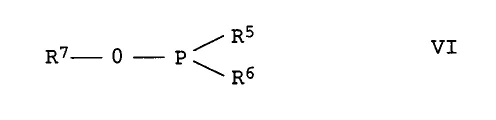 zu den Arenbisphosphinoxiden, wobei die Reste R¹ bis R⁶ die oben genannte Bedeutung haben und R⁷ für eine C₁-C₆-Alkylgruppe, eine C₁-C₆-Cycloalkylgruppe oder eine C₁-C₆-Alkoxygruppe steht.

In den Arenbisphosphinoxiden der allgemeinen Formel I stehen die Variablen R¹ bis R⁴ bevorzugt unabhängig voneinander für ein Wasserstoffatom, eine C₁- bis C₆-Alkyl- oder -Alkoxygruppe. Besonders bevorzugt stehen R¹ bis R⁴ für ein Wasserstoffatom oder eine C₁- bis C₄-Alkylgruppe. Insbesondere leiten sich die Arenbisphosphinoxide von Mesitylen ab, so daß einer der Reste R¹ bis R⁴ für ein Wasserstoffatom steht und die verbleibenden Reste für C₁- bis C₄-Alkylgruppen, vorzugsweise Methylgruppen, stehen, in der Weise, daß die C₁- bis C₄-Alkylgruppen jeweils zueinander in meta-Position am aromatischen Ring stehen.

Als Beispiel für die Reste R¹ bis R⁴ seien neben Wasserstoff die Methyl-, Ethyl-, i-Propyl-, n-Propyl, n-Butyl-, Amyl-, n-Hexyl-, Cyclopentyl-, Cyclohexyl-, Methoxi-, Ethoxi-, Propoxi-, i-Propoxi-, Butoxi-, Ethoxylethoxi- oder eine Aryloxigruppe, wie die Phenoxi-, Methylpehnyloxi-, Benzyloxigruppe, genannt.

Die Variablen R⁵ und R⁶ stehen bevorzugt unabhängig voneinander für eine C₁-C₆-Alkyl- oder -Alkoxygruppe oder eine C₅-C₁₂-Arylgruppe. Besonders bevorzugt stehen R⁵ und R⁶ für eine C₁-C₄-Alkoxygruppe oder einen Phenylring.

Als mögliche Reste R⁵ oder R⁶ seien z.B. die Ethyl-, i-Propyl-. n-Propyl-, n-Butyl-, Amyl-, n-Hexyl-, Cyclopentyl-, Cyclohexylgruppe, eine Arylgruppe, wie die Phenyl-, Naphthylgruppe, eine halogensubstituierte Arylgruppe, wie die Mono- oder Dichlorphenylgruppe, eine alkylsubstituierte Phenylgruppe, wie die Methylphenyl-, Ethylphenyl-, Isopropylphenyl-, t-Butylphenyl-, Dimethylphenylgruppe, eine alkoxisubstituierte Arylgruppe, wie die Methoxiphenyl-, Ethoxiphenyl-, Dimethoxiphenylgruppe, S- oder N-haltige fünf- oder sechsgliedrige Ringe, wie die Thiophenyl-, Pyridylgruppe oder eine Alkoxygruppe, wie die Methoxi-, Ethoxi-, i-Propoxi-, Butoxi-, Ethyloxiethoxigruppe, eine Aryloxigruppe, wie die Phenoxi-, Methylphenyloxi- oder Benzyloxigruppe genannt.

Als Arenbisphosphinoxide genannt seien z.B.
1,3-Bis(diphenylphosphonocarbonyl)benzol,
1,3-Bis(diethoxiphosphonocarbonyl)benzol,
1,3-Bis(ethoxiphenylphosphonocarbonyl)benzol,
oder die entsprechenden 1,2 oder 1,4 substituierten Derivate,
1,3-Bis(diphenylphosphonocarbonyl)2,4,6-trimethoxibenzol
1,3-Bis(diethoxyphosphonocarbonyl)2,4,6-trimethoxibenzol
1,3-Bis(ethoxiphenylphosphonocarbonyl)2,4,6-trimethoxibenzol,
1,3-Bis(diphenylphosphonocarbonyl)2,4,6-trichlorbenzol,
1,3-Bis(diethoxiphosphonocarbonyl)2,4,6-trichlorbenzol,
1,3-Bis(ethoxiphenylphosphonocarbonyl)2,4,6-trichlorbenzol,
oder Verbindungen der Strukturen 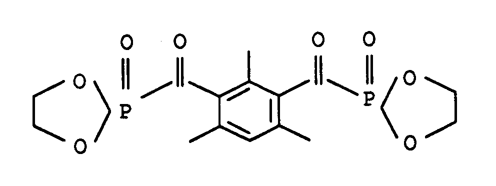 oder

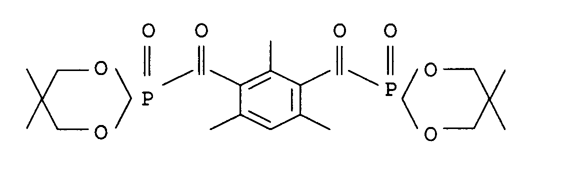

Bei der Herstellung der Arenbisphosphinoxide geht man aus von Verbindungen der Formel

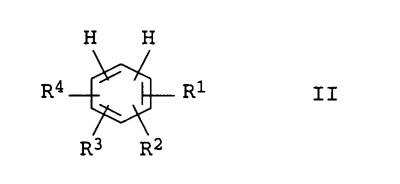

Die Reste R¹ - R⁴ haben die oben genannte Bedeutung. Ganz besonders bevorzugt handelt es sich bei der Verbindung II um Mesitylen (1,3,5 Trimethylbenzol).

Die Verbindung der Formel II wird durch Umsetzung mit α-Halogenacetylhalogeniden, insbesondere α-Halogenacetylchloriden, z.B. Chloracetylchlorid oder Bromacetylchlorid in Gegenwart von Eisen-III-oxid als Katalysator bei Temperaturen zwischen 0 und 150°C, bevorzugt zwischen 50 und 100°C und Drücken zwischen 0,01 und 50 bar, bevorzugt zwischen 0,5 und 5 bar in das Bishalogenacetophenon der Formel 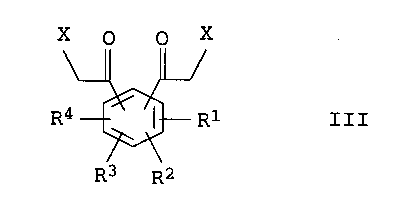 überführt. Die Reaktion kann in Gegenwart eines Lösungsmittels oder auch lösungsmittelfrei durchgeführt werden. Geeignete Lösungsmittel sind z.B. aromatische Verbindungen wie Nitrobenzol oder Chlorbenzol, halogenierte Alkane wie Dichlorethan oder Dichlormethan oder Alkane wie Hexan oder Heptan.

Zur zweifachen Halogenacetylierung werden mindestens 2 Mol α-Halogenacetylhalogenid, bezogen auf 1 Mol der Verbindung II, eingesetzt.

Die Ausgangskomponenten werden vorzugsweise im Molverhältnis 2:1 bis 20:1 (α-Halogenacetylhalogenid: Verbindung II), besonders bevorzugt in stöchiometrischen Verhältnissen, eingesetzt.

Die erhaltene Verbindung III wird durch eine Haloformspaltung zur Dicarbonsäure der Formel 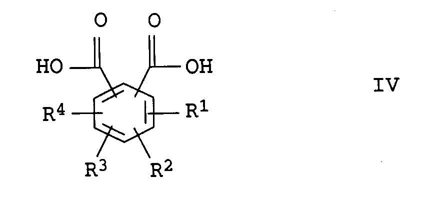 umgesetzt.

Die Haloformspaltung der Verbindung III wird vorzugsweise durchgeführt in einer Alkalihypohalogenidlösung in Gegenwart eines Phasentransferkatalysators bei Temperaturen von 0 bis 150°C, bevorzugt 30 bis 80°C, besonders bevorzugt 40 bis 70°C und Drücken von 0,01 bis 50 bar, bevorzugt 0,1 bis 5 bar, besonders bevorzugt bei Normaldruck. Die Alkalihypohalogenidlösung kann als solche eingesetzt werden; sie kann aber auch während der Reaktion in situ erzeugt werden aus einer Alkalyhydroxidlösung und einem Halogen wie Chlor oder Brom, bevorzugt Chlor.

Als Alkalihydroxide eignen sich Lithiumhydroxid, Natriumhydroxid, Kaliumjydroxid, Rubidiumhydroxid und Cäsiumhydroxid, besonders bevorzugt Natriumhydroxid und Kaliumhydroxid. Das Molverhältnis von Alkalihypohalogenid zu Verbindung III kann in weiten Grenzen variiert werden, liegt in der Regel bei 0,8:1 bis 50:1, bevorzugt 1:1 bis 20:1, besonders bevorzugt 1:1 bis 5:1.

Als Phasentransferkatalysatoren kommen zweckmäßig quarternäre Salze in Betracht, insbesondere Katalysatoren der Formel 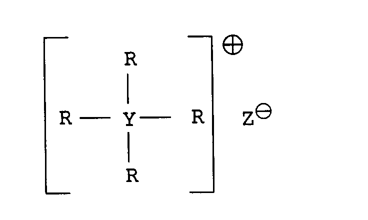 worin die einzelnen Rest R gleich oder verschieden sein können und unabhängig voneinander für einen aliphatischen, cycloaliphatischen, aromatischen, araliphatischen Rest stehen, Y ein Stickstoff-, Phosphor- oder Arsenatom bezeichnet und Z für ein Anion steht. In bevorzugten Katalysatoren steht R für eine Alkylgruppe oder eine Alkoxigruppe mit jeweils 1 bis 18, insbesondere 1 bis 7 Kohlenstoffatomen, eine Cycloalkylgruppe mit 5 bis 8 Kohlenstoffatomen, eine Aralkylgruppe oder eine Alkylarylgruppe mit 7 bis 12 Kohlenstoffatomen oder eine Phenylgruppe.

Das Säureanion Z^{⊖} kann sich z.B. von Mineralsäuren wie Salzsäure, Schwefelsäure, Phosphorsäure, Sulfonsäuren wie Benzol- und p-Toluolsulfonsäure oder von Carbonsäuren, insbesondere der Mono-, Di- oder Trichloressigsäure, ableiten.

Die Dicarbonsäure wird anschließend in das Bissäurechlorid der Formel 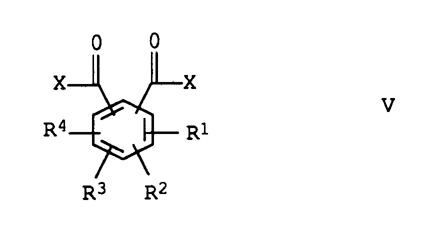 überführt, was durch übliche Umsetzung mit Thionylchlorid erfolgen kann.

Schließlich werden die Arenbisphosphinoxide erhalten durch Umsetzung mit Phosphinen der allgemeinen Formel 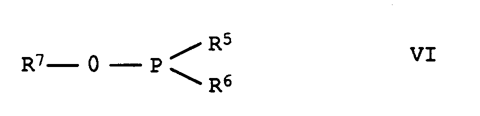 worin R⁵ und R⁶ die oben genannte Bedeutung haben und R⁷ für eine C₁-C₆-Alkylgruppe, eine C₅-C₁₀-Cycloalkylgruppe oder eine C₁-C₆-Alkoxygruppe steht, erhalten.

Geeignete Phosphine sind zum Beispiel Methyldimethoxiphosphin, Butyldimethoxiphosphin, Phenyldimethoxiphosphin, Tolyldimethoxiphosphin, Phenyldiethoxiphosphin, Tolyldiethoxiphosphin, Phenyldibutoxiphosphin, Tolyldibutoxiphosphin bzw. Dimethylmethoxiphosphin, Dibutylmethoxiphosphin, Dimethylbutoxiphosphin, Diphenylmethoxiphosphin, Diphenylethoxiphosphin, Diphenylpropoxiphosphin, Diphenylisopropoxiphosphin, Diphenylbutoxiphosphin bzw. Trimethylphosphit, Triethylphosphit, Tripropylphosphit, Triisopropylphosphit, Tributylphosphit

Die Umsetzung zwischen dem Bissäurechlorid und dem Phosphin kann in einem Lösungsmittel, z.B. einem Kohlenwasserstoff oder Kohlenwasserstoffgemisch, wie Petrolether, Toluol, Cyclohexean, einem Ether, anderen üblichen inerten organischen Lösungsmitteln oder auch ohne Lösungsmittel bei Temperaturen zwischen -30°C und +150°C, bevorzugt bei 10°C bis 100°C, ausgeführt werden. Das Produkt kann aus dem Lösungsmittel direkt auskristallisiert werden oder hinterbleibt nach dem Abdampfen.

Bei der Umsetzung können die Ausgangsstoffe in stöchiometrischen Verhältnissen eingesetzt werden, da ein Überschuß eines der Ausgangskomponenten aufgrund des guten Umsatzes im allgemeinen nicht notwendig ist.

Die Arenbisphosphinoxide können als Photoinitiator in photopolymerisierbaren Massen eingesetzt werden.

Solche photopolymerisierbaren bzw. strahlungshärtbaren Massen enthalten photopolymerisierbare ethylenisch ungesättigte Verbindungen.

Als photopolymerisierbare Monomere eignen sich Verbindungen mit polymerisierbaren C-C-Doppelbindungen, die z.B. durch Aryl-, Carbonyl-, Amino-, Amido-, Ester-, Carboxi- oder Cyanid-Gruppen, Halogenatome oder weitere C-C-Doppel- oder C-C-Dreifachbindungen aktiviert sind. Genannt seien beispielsweise Vinylether und Vinylester, Styrol, Vinyltoluol, Arylsäure und Methacrylsäure sowie deren Ester mit ein- und mehrwertigen Alkoholen, deren Nitrile oder Amide, Malein- und Fumarsäureester sowie N-Vinylpyrrolidon, N-Vinylcaprolactam, N-Vinylcarbazol und Allylester wie Diallylphthalat.

Als photopolymerisierbare höhermolekulare Verbindungen seien beispielsweise ungesättigte Polyester auf Basis von α,β-ungesättigten Dicarbonsäuren wie Maleinsäure, Fumarsäure oder Itaconsäure, gegebenenfalls im Gemisch mit gesättigten, beziehungsweise aromatischen Dicarbonsäuren wie Adipinsäure, Phthalsäure, Tetrahydrophthalsäure oder Terephthalsäure und Alkandiolen wie Ethylenglykol, Propylenglykol, Butandiol, Neopentylglykol oder oxalkyliertem Bisphenol-A genannt.

Weiterhin von Bedeutung sind (Poly)Epoxid(meth)acrylate, wie sie durch Umsetzung von Acrylsäure oder Methacrylsäure mit Diglycidylethern erhältlich sind, Polyester(meth)acrylate oder Urethan(meth)acrylate.

Die photopolymersierbaren Massen enthalten die Verbindungen im allgemeinen in einer Konzentration von 0,001 bis 20 Gew.-%, bevorzugt 0,1 bis 5 Gew.-%, bezogen auf die polymerisierbare Masse.

Die Arenbisphosphinoxide können mit Beschleunigern kombiniert werden, die einem gegebenenfalls hemmenden Einfluß des Luftsauerstoffs auf die Photopolymerisation entgegenwirken.

Solche Beschleuniger beziehungsweise Synergisten sind beispielsweise sekundäre und/oder tertiäre Amine wie Methyldiethanolamin, Dimethylethanolamin, Triethylamin, Triethanolamin, p-Dimethylaminobenzoesäureethylester, Benzyldimethylamin, Dimethylaminoethylacrylat, N-Phenylglycin, N-Methylglycin und analoge, dem Fachmann bekannte Verbindungen. Zur Beschleuinigung der Aushärtung können weiterhin aliphatische und aromatische Halogenide dienen, wie 2-Chlormethylnaphtalin, 1-Chlor-2-chlormethylnaphthalin oder Radikalbildner wie Peroxide und Azo-Verbindungen.

Es können auch Mischungen der Arenbisphosphinoxide mit bereits bekannten Photoinitiatoren eingesetzt werden. Bei Einsatz solcher Mischungen sind zum Teil synergistische Effekte zu beobachten.

Besonders wirksame synergistische Mischungen ergeben sich bei Kombination mit bekannten Photoinitiatoren auf Basis aromatischer Ketone, insbesondere Benzildimethylketal, Hydroxiisobutyrophenon, Diethoxiacetophenon, Benzophenon, und 2-Methylthioxanthon, 2-Isopropylthioxanthon sowie 2-Chlor-thioxanthon.

Die photopolymerisierbaren Massen, welche die Arenbisphosphinoxide enthalten, weisen eine sehr gute Lagerstabilität auf.

Die photopolymerisierbaren Massen können als Beschichtungsmassen, z.B. als Lack oder Druckfarbe oder zur Herstellung von Formkörpern, z.B. Druckplatten, Photoresisten oder Reliefformen, verwendet werden.

Des weiteren können die photopolymerisierbaren Massen als Füll- oder Spachtelmassen Verwendung finden. Für diese Verwendung sind insbesondere auch die oben genannten Massen auf Basis ungesättigter Polyester oder (Poly)Epoxid(meth)acrylate geeignet.

Eine weitere Verwendungsmöglichkeit sind Dentalmassen, z.B. als Zahnfüllmassen oder Zahnersatzkörper.

Bei den photopolymerisierbaren Massen kann es sich auch um Lösungen oder Dispersionen, insbesondere wäßrige Dispersionen, handeln, welche photopolymerisierbare Verbindungen enthalten. Die photopolymerisierbaren Massen können jeweils für die beabsichtigte Verwendung übliche Additive, z.B. Farbstoffe oder Pigmente bei der Verwendung als Lack enthalten.

Als weitere Additive genannt seien z.B. Inhibitoren gegen die thermische Polymerisation, Verlaufshilfsmittel, Füllstoffe und Mattierungsmittel sowie Stabilisatoren gegen thermischen oder photochemischen Abbau.

Als Strahlungsquellen für das die Polymerisation solcher Mischungen auslösende Licht verwendet man solche, die Licht vorzugsweise im Adsorptionsbereich der erfindungsgemäßen Verbindungen aussenden, d.h. insbesondere zwischen 230 und 450 mm. Besonders geeignet sind Quecksilber-Niederdruckstrahler, -Mitteldruckstrahler und Hochdruckstrahler sowie Leuchtstoffröhren oder Impulsstrahler.

Mit den Photoinitiatoren lassen sich photopolymerisierbare Massen, insbesondere auch weißpigmentierte Lacke, vergilbungsfrei härten. Des weiteren zeichnen sich die Photoinitiatoren durch ihre hohe Reaktivität aus. Die Härtungszeiten der photopolymerisierbaren Massen sind durch die hohe Reaktivität der Arenbisphosphinoxide erheblich verkürzt.

### Beispiele

### I. Herstellung der Arenbisphosphinoxide

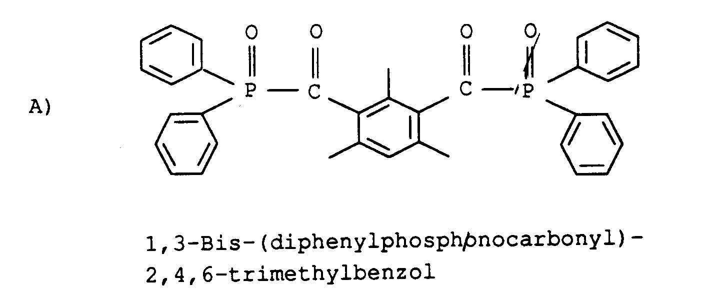

240 g 1,3,5-Trimethylbenzol und 160 mg Fe₂O₃ wurden in der 1. Stufe vorgelegt und auf 80°C aufgeheizt. Innerhalb einer Stunde wurden insgesamt 503 g Chloracetylchlorid zugegeben. Kurz nach Beginn der Zugabe löste sich der Katalysator Fe₂O₃ mit roter Farbe vollständig auf, gleichzeitig setzt HCl-Entwicklung ein. Nachdem 50 % der theoretischen HCl-Menge frei geworden waren, wurde begonnen, Stickstoff durch das Reaktionsgemisch zu leiten.

Nach beendeter Gasentwicklung wurde der Reaktionsaustrag mit 5 g Wasser versetzt und im Vakuum destilliert. Das Produkt 1,3-Bis-(-chloracetyl)-2,4,6-trimethylbenzol hatte einen Siedepunkt von 140°C bei 0,3 mbar, Schmelzpunkt: 129 - 131°C, Ausbeute: 376 g.

In der 2. Stufe wurden 500 g Natriumhypochloridlösung (13 Gew.-% aktives Chlor), 720 g Natronlauge (25 %ig) und 4 g Dimethyldibenzylammoniumchlorid als 50 %ige wäßrige Lösung vorgelegt und auf 50°C aufgeheizt. Man gab anschließend 85 g 1,3-Bis-(chloracetyl)-2,4,6-trimethylbenzol zu. Innerhalb einer Stunde wurden noch 66 g Chlorgas eingeleitet und danach für 5 Stunden bei Rückflußtemperatur (ca. 105 - 108°C) gerührt.

Nach dem Abkühlen auf Raumtemperatur wurde die Reaktionslösung abgelassen und mit ca. 200 ml konzentrierter Salzsäure auf pH = 2 eingestellt. Die weiße Suspension wurde abfiltriert, der Niederschlag zweimal mit je 250 ml Wasser gewaschen und bei 80°C im Vakuum getrocknet.

Man erhielt 61,5 g 2,4,6.Trimethylbenzol-1,3-dicarbonsäure.

In der 3. Stufe wurden 85 g Thionylchlorid vorgelegt und auf 40°C aufgeheizt. Dazu wurden innerhalb von 45 min 57,3 g 2,4,6-Trimethylbenzol-1,3-dicarbonsäure gegeben. Man rührte bei 40 - 50°C bis zum Ende der Gasentwicklung nach.

Der Rückstand wurde im Vakuum destilliert und es wurden 38 g 2,4,6-Trimethylbenzol-1,3-dicarbonsäuredichlorid mit einem Siedepunkt von 113°C bei 1,4 mbar erhalten.

In der 4. Stufe wurden 34,3 g 2,4,6-Trimethylbenzol-1,3-dicarbonsäuredichlorid bei 80°C vorgelegt. Innerhalb einer Stunde gab man 64,4 g Ethoxidiphenylphosphin zu und rührte noch eine Stunde bei dieser Temperatur nach. Danach wurden nacheinander 50 ml Xylol und 50 ml Testbenzin zugegeben. Das Produkt kristallisierte beim langsamen Abkühlen aus, wurde abgesaugt und im Vakuum getrocknet.

Die Ausbeute betrug 75 g 1,3-Bis-(diphenylphosphinocarbonyl) 2,4,6-trimethylbenzol (Schmelzpunkt 164 - 165°C).

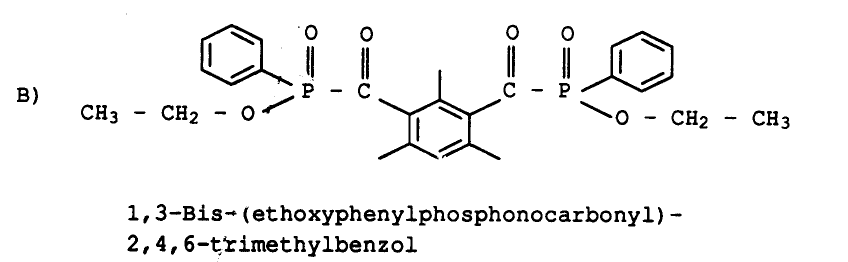

Die Herstellung in den ersten drei Stufen wurde wie bei A) beschrieben durchgeführt. In der 4. Stufe wurden 53 g 2,4,6-Trimethylbenzol-1,3-dicarbonsäuredichlorid und 74 g Diethoxiphenylphosphin analog Stufe 4 unter A bei 80°C umgesetzt. Es wurden 107 g flüssiges Endprodukt erhalten.

### II. Anwendungstechnische Prüfungen

### Bestimmung der Reaktivität

In 96 g eines Oligoetheracrylats auf der Basis eines Umsetzungsprodukts von alkoxyliertem Trimethylolpropan mit Acrylsäure (Laromer® LR 8812 von BASF) wurden 4 g des unten aufgeführten Photoinitiators gelöst. Die photopolymerisierbare Masse wurde in einer Schichtstärke von 50 g/m2 auf Kontrastpapier aufgetragen und in 30 cm Abstand auf einem Transportband unter einem Quecksilberhochdruckstrahler (120 W/cm) vorbeigeführt.

Die maximal mögliche Geschwindigkeit des Transportbandes, bei der noch eine Aushärtung erfolgt, gilt als Maß für die Reaktivität des Photoinitiators.

| Photoinitiator | maximal mögliche Geschwindigkeit des Transportbandes [m/min] |
|---|---|
| aus Beisp. I A | 20 |
| aus Beisp. I B | 20 |
| zum Vergleich 2,4,6-Trimethylbenzoyldiphenylphosphinoxid (Lucirin® TPO der BASF) | 16 |

## Patentansprüche

1. Verfahren zur Herstellung von Arenbisphosphinoxiden der allgemeinen Formel I, 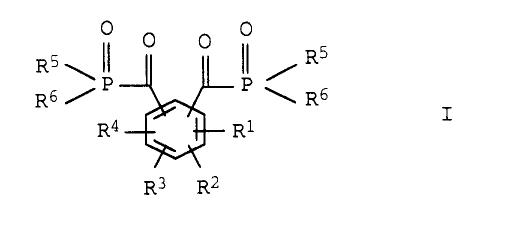 worin die Reste R¹ bis R⁴ unabhängig voneinander für ein Wasserstoffatom oder eine C₁-C₆-Alkyl-, -Alkoxygruppe oder eine C₅-C₁₂-Aryl-, eine C₅-C₁₂-Aryloxy- oder eine C₇-C₁₄-Arylalkoxygruppe stehen, R⁵ und R⁶ unabhängig voneinander für eine C₁-C₆-Alkylgruppe, eine C₅-C₁₀-Cycloalkylgruppe oder eine C₅-C₁₂-Arylgruppe, welche auch ein oder zwei Stickstoff- oder Schwefelatome im Ringsystem enthalten oder ein oder zwei Halogenatome, C₁-C₆-Alkyl- oder -Alkoxygruppen als Substituenten am Ringsystem tragen können, oder für eine C₁-c₆-Alkoxy-, C₃-C₈-Alkyloxyalkoxy-, eine C₅-C₁₂-Aryloxy- oder C₇-C₁₄-Arylalkoxygruppe stehen können oder R⁵ und R⁶ gemeinsam eine Brücke aus 2 bis 10 Kohlenstoffatomen bilden, wobei auch ein Teil der Kohlenstoffatome Bestandteil eines aromatischen Rings sein kann, gekennzeichnet durch folgende Verfahrensschritte:
a) Zweifache Halogenacetylierung einer Verbindung der Formel 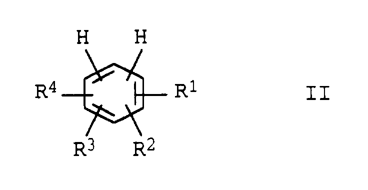 mit α-Halogenacetylhalogeniden in Gegenwart von Eisen-III-oxid.
b) Überführung des in a) erhaltenen Bishalogenacetophenon der Formel 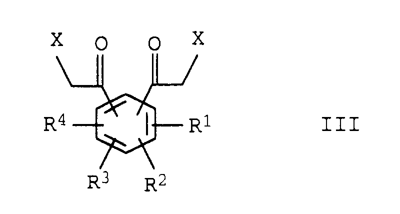 durch Umsetzung mit einer Alkalihypohalogenidlösung in Gegenwart eines Phasentransferkatalysators in die Dicarbonsäure der Formel 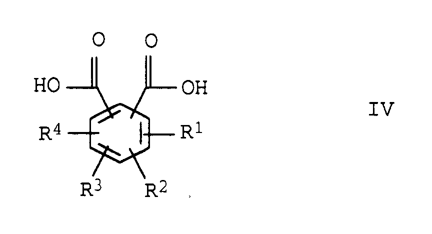
c) Überführung der Dicarbonsäure in das entsprechende Bissäurechlorid der Formel 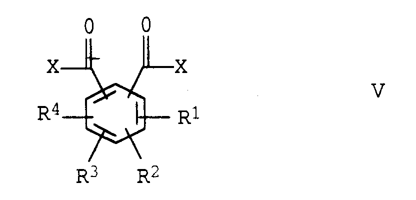 und
d) Umsetzung des Bissäurechlorids mit Phosphinen der Formel 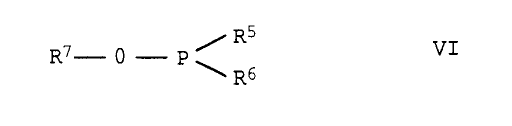 zu den Arenbisphosphinoxiden, wobei die Reste R¹ bis R⁶ die oben genannte Bedeutung haben und R⁷ für eine C₁-C₆-Alkylgruppe, eine C₁-C₆-Cycloalkylgruppe oder eine C₁-C₆-Alkoxygruppe steht.

2. Verfahren gemäß Anspruch 1, dadurch gekennzeichnet, daß die Reste R¹ bis R⁴ unabhängig voneinander für ein Wasserstoffatom oder eine C₁-C₄ Alkylgruppe stehen und R⁵ und R⁶ unabhängig voneinander für einen Phenylring oder eine C₁-C₄ Alkoxygruppe stehen.

3. Verfahren gemäß Anspruch 1 oder 2, dadurch gekennzeichnet, daß einer der Reste R¹ bis R⁴ für ein Wasserstoffatom steht und die verbleibenden Reste R¹ bis R⁴ für C₁-C₄ Alkylgruppen stehen, welche am aromatischen Ring jeweils zueinander in meta-Position substituiert sind.

4. Verfahren nach Anspruch 1, dadurch gekennzeichnet, daß es sich bei der Verbindung der allgemeinen Formel II um Mesitylen handelt.

## Claims

1. A process for the preparation of an arenebisphosphine oxide of the formula I, 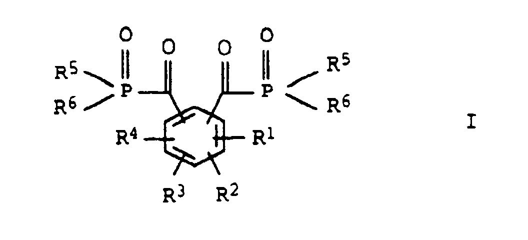 where R¹ to R⁴ independently of one another are each hydrogen, C₁-C₆-alkyl, C₁-C₆-alkoxy, C₅-C₁₂-aryl, C₅-C₁₂-aryloxy or C₇-C₁₄-arylalkoxy, R⁵ and R⁶ independently of one another may each be C₁-C₆-alkyl, C₅-C₁₀-cycloalkyl or C₅-C₁₂-aryl, each of which may furthermore contain one or two nitrogen or sulfur atoms in the ring system or carry one or two halogen atoms or C₁-C₆-alkyl or C₁-C₆-alkoxy groups as substituent on the ring system, or are each C₁-C₆-alkoxy, C₃-C₈-alkoxyalkoxy, C₅-C₁₂-aryloxy or C₇-C₁₄-arylalkoxy, or R⁵ and R⁶ together form a bridge of 2 to 10 carbon atoms, in which some of the carbon atoms may furthermore be part of an aromatic ring, which comprises the following process steps:
a) introduction of two haloacetyl groups into a compound of the formula 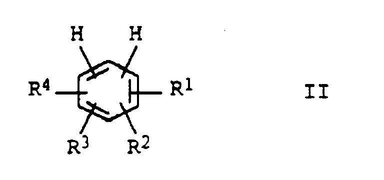 by means of an α-haloacetyl halide in the presence of iron (III) oxide,
b) conversion of the bishaloacetophenone obtained in a) of the formula 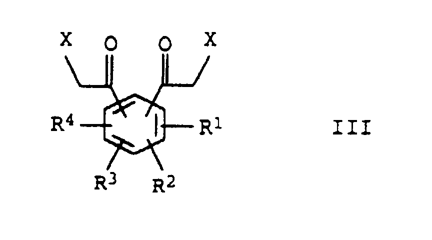 into the dicarboxylic acid of the formula 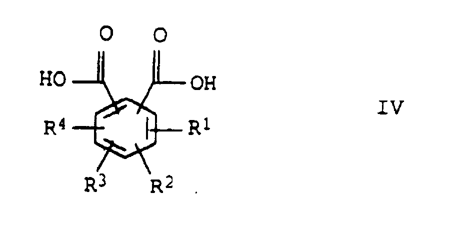 by reaction with an alkali metal hypohalite solution in the presence of a phase transfer catalyst,
c) conversion of the dicarboxylic acid into the corresponding bisacyl chloride of the formula 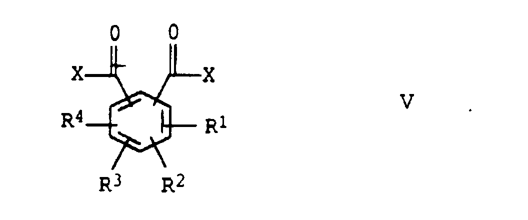 and
d) reaction of the bisacyl chloride with a phosphine of the formula 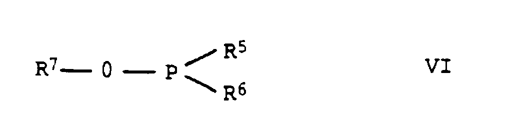 to give the arenebisphosphine oxide in which R¹ to R⁶ have the abovementioned meanings and R⁷ is a C₁-C₆-alkyl, C₁-C₆-cycloalkyl or C₁-C₆-alkoxy group.

2. A process as claimed in claim 1, wherein R¹ to R⁴ independently of one another are each hydrogen or C₁-C₄-alkyl and R⁵ and R⁶ independently of one another are each phenyl or C₁-C₄-alkoxy.

3. A process as claimed in claim 1 or 2, wherein one of the radicals R¹ to R⁴ is hydrogen and the remaining radicals R¹ to R⁴ are each C₁-C₄-alkyl groups which are in the meta position relative to one another on the aromatic ring.

4. A process as claimed in claim 1, wherein the compound of the formula II is mesitylene.

## Revendications

1. Procédé de préparation de bisphosphinoxydes d'arène de formule générale I 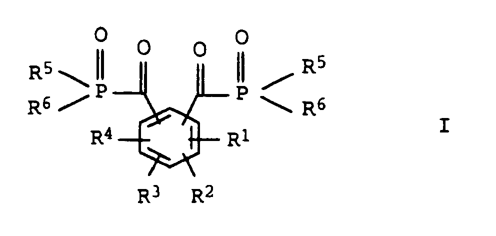 dans laquelle les restes R¹ à R⁴ représentent indépendamment les uns des autres un atome d'hydrogène ou un groupement alkyle en C₁-C₆ ou alcoxy en C₁-C₆ ou un groupement aryle en C₅-C₁₂, aryloxy en C₅-C₁₂ ou (aryle en C₇-C₁₄)alcoxy, R⁵ et R⁶ représentent indépendamment les uns des autres un groupement alkyle en C₁-C₆ cycloalkyle en C₅-C₁₀ ou aryle en C₅-C₁₂, pouvant contenir aussi un ou deux atomes d'azote ou de soufre dans le cycle ou pouvant porter en tant que substituants du cycle, un ou deux atomes d'halogène, groupements alkyle en C₁-C₆ ou alcoxy en C₁-C₆, ou bien représentent un groupement alcoxy en C₁-C₆, (alkyle en C₃-C₈)oxyalcoxy, aryloxy en C₅-C₁₂ ou (aryle en C₇-C₁₄)alcoxy ou bien R⁵ et R⁶ forment ensemble un pont de 2 à 10 atomes de carbone, une partie des atomes de carbone pouvant également faire partie d'un cycle aromatique, caractérisé par les étapes de procédé suivantes :
a) double halogéno-acétylation d'un composé de formule 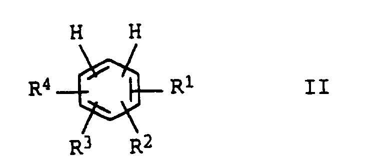 avec des halogénures d'α-halogénoacétyle en présence d'oxyde de fer-III,
b) conversion de la bishalogénoacétophénone obtenue en a) de formule 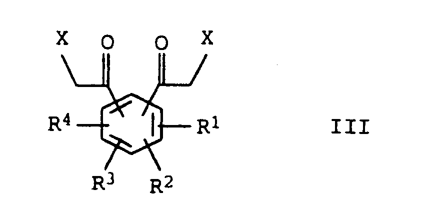 par réaction avec une solution d'hypohalogénure d'alcali en présence d'un catalyseur de transfert de phases en l'acide dicarboxylique de formule 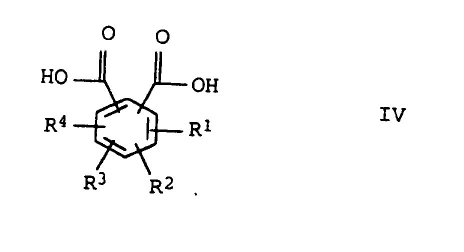
c) conversion de l'acide dicarboxylique en le bis(chlorure d'acide) correspondant de formule 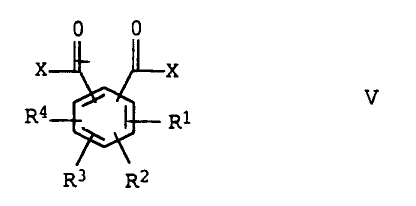 et
d) réaction du bis(chlorure d'acide) avec des phosphines de formule 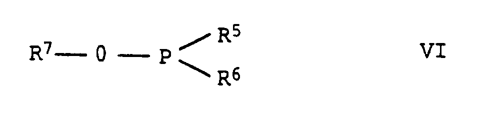 pour donner des bisphosphinoxydes d'arène, où les restes R¹ à R⁶ prennent la signification mentionnée au-dessus et R⁷ est mis pour un groupement alkyle en C₁-C₆, cycloalkyle en C₁-C₆ ou alcoxy en C₁-C₆.

2. Procédé selon la revendication 1, caractérisé en ce que les restes R¹ à R⁴ sont mis indépendamment les uns des autres pour un atome d'hydrogène ou un groupement alkyle en C₁-C₄ et R⁵ et R⁶ sont mis indépendamment les uns des autres pour un cycle phényle ou un groupement alcoxy en C₁-C₄.

3. Procédé selon la revendication 1 ou 2, caractérisé en ce que l'un des restes R¹ à R⁴ est mis pour un atome d'hydrogène et les restes R¹ à R⁴ restants sont mis pour des groupements alkyle en C₁-C₄ qui sont chacun substitués sur le cycle aromatique en position méta les uns par rapport aux autres.

4. Procédé selon la revendication 1, caractérisé en ce que le composé de formule générale II est le mésitylène.
